Europäisches Patentamt

European Patent Office

Office européen des brevets

(11) Veröffentlichungsnummer: **0 218 957 B1**

## EUROPÄISCHE PATENTSCHRIFT

(45) Veröffentlichungstag der Patentschrift: **10.06.92**

(21) Anmeldenummer: **86113101.9**

(22) Anmeldetag: **24.09.86**

(51) Int. Cl.⁵: **A61B 5/103**, A61B 5/08

(54) **Vorrichtung zur Überwachung der Atmung und Herztätigkeit.**

(30) Priorität: **12.10.85 DE 3536491**

(43) Veröffentlichungstag der Anmeldung:
**22.04.87 Patentblatt 87/17**

(45) Bekanntmachung des Hinweises auf die
Patenterteilung:
**10.06.92 Patentblatt 92/24**

(84) Benannte Vertragsstaaten:
**AT BE CH DE FR GB IT LI LU NL SE**

(56) Entgegenhaltungen:
**CH-A- 410 274**
**US-A- 3 890 958**

**ELECTRO MEDICA (SIEMENS), Band 49, Nr. 4, 1981, Seiten 194-199, Erlangen, DE; K. RICHTER et al.: "Über eine neue Möglichkeit zur Erfassung der Atemtätigkeit von Früh- und Neugeborenen durch Registrierung des Mechanogramms"**

**BIOMEDIZINISCHE TECHNIK, Band 25, Nr. 7/8, Juli/August 1980, Seiten 186-192, Berlin, DE; H. SCHWERDT et al.: "Ein neuer Vektor-Ballistokardiograph mit direkter Kraftmessung"**

**SIEMENS FORSCHUNGS- UND ENTWICKLUNGSBERICHTE, Band 10, Nr. 2, 1981, Seiten 104-109, Würzberg, DE; H. REICHENBERGER et al.: "Indirekte Erfassung mechanischer Lebensfunktionen von Säuglingen"**

(73) Patentinhaber: **Morgenstern, Jürgen, Prof. Dr.**
**Im Heidewinkel 33**
**W-4000 Düsseldorf(DE)**

(72) Erfinder: **Morgenstern, Jürgen, Prof. Dr.**
**Im Heidewinkel 33**
**W-4000 Düsseldorf(DE)**

(74) Vertreter: **Plöger, Ulrich, Dipl.-Ing.**
**Benrather Schlossallee 89**
**W-4000 Düsseldorf-Benrath(DE)**

EP 0 218 957 B1

## Beschreibung

Vorrichtung zur Überwachung der Atmung und Herztätigkeit bei einer in einem Bett ruhenden Person, insbesondere bei Kleinkindern, wobei von der zu überwachenden Person ausgehende Impulse auf einen in vertikaler Richtung ausgerichteten Sensor einwirken.

Eine derartige Vorrichtung ist in der Dissertation "Über eine indirekte Methode zur kontinuierlichen Atmungsregistrierung" von H. U. Thal, Universität Düsseldorf, 1973, beschrieben. Danach werden piezo-elektrische Lastzellen in den fußseitigen Beinen von Betten eingebaut. Es kommt beim Ein- und Ausatmen eines Patienten zu einer Schwerpunktsverlagerung, so daß die Bettfüße demgemäß eine unterschiedliche Belastung erfahren. Diese Belastungen werden mittels der Lastzellen aufgenommen und nach Durchgang durch einen Verstärker als Signal dargestellt. Man gewinnt hierbei zugleich ein sogenanntes Ballistocardiagramm, welches mit entsprechender Frequenz für die Herztätigkeit charakteristisch ist, sowie eine Atemkurve, die mit niedrigerer Frequenz die Atmungsreaktionen aufzeichnet. Die durch die Herztätigkeit ausgelösten Signale werden in diesem Falle mittels eines entsprechenden Filters ausgeschieden, um eine eindeutige Aussage über den Atmungsverlauf zu gewinnen. Von erheblicher Bedeutung ist diese Art der Überwachung nicht nur in der Wachstation einer Klinik, sondern auch in der Neugeborenen-Station, weil auf diesem Wege durch entsprechende Signalverwertung Anzeichen eines Atmungsstillstandes sofort erkannt werden können, so daß die erforderlichen Gegenmaßnahmen eingeleitet werden können. Der Vorteil dieses Verfahrens besteht darin,daß eine Überwachung der Atemtätigkeit möglich ist, ohne beim Patienten bzw. Neugeborenen selbst irgendwelche Messungen vornehmen zu müssen. Indes sind weitergehende Möglichkeiten der Überwachung auf dieser Grundlage weder vorgesehen noch möglich.

Der Erfindung liegt demgegenüber die Aufgabenstellung zugrunde, zu einer weitergehenden Aussage der Atmungs- und Herztätigkeit zu gelangen, als dies mit der bekannten Vorrichtung möglich ist. Es soll somit zumindest neben einer Aussage über die Atmungstätigkeit laufend auch über die Herztätigkeit auf der Grundlage zuverlässig verwertbarer Signale ermöglicht werden. Die Aussagemöglichkeiten sollen darüber hinaus in Richtung einer weitergehend differenzierten Überwachung sowohl der Atmung als auch der Herztätigkeit ausgedehnt werden. Bei der Atmung sind noch zusätzliche Angaben über die Atmungsart (Bauch- oder Brustatmung) sowie den Atmungsdruck von Interesse, während für die Herztätigkeit die Richtung des Blutauswurfs besonders bei Neugeborenen erhöhte Beachtung verdient. So würde der Blutauswurf in einen noch offenen ductus Botalli einen charakeristisch gerichteten Impuls ergeben, der zur Verbindung der arteria pulmonalis mit der aorta descendens einen Winkel unter 90° bildet. In der Rück- bzw. Bauchlage und in der Links- bzw. Rechtsseitenlage würden sich demgemäß unterschiedliche Impulskomponenten ergeben.

Die Erfindung löst die vorerwähnte Aufgabenstellung durch die in den Patentansprüchen gemachten Vorschläge.

Somit ist nach einem ersten Merkmal der Erfindung zunächst die Möglichkeit einer Reaktionsmöglichkeit des Bettes auf weiterreichende Impulse als die bisher lediglich erfaßte Massenverschiebung und demgemäße Schwerpunktsverlagerung gegeben. Impulse sind in diesem Zusammenhang im allgemeinen Sinne kurzzeitige Wirkungen mechanischer Größen bzw. ihre kurzzeitige Abweichung von einem Grundwert. Daher kommt man nicht nur zu einer richtungsabhängigen Aussage über die Massenverschiebungen beim Atmen, sondern auch über die damit verbundenen Reaktionskraftverläufe beim Einatmen und beim Ausatmen. Da das Bett nicht nur allseitig beweglich gelagert ist, sondern auch einer Rückstellkraft unterliegt, kommt es auf das Ausmaß, in welchem Lageänderungen des Bettes tatsächlich eintreten, weniger an. Indes besteht für die Untersuchung der zu erfassenden Vorgänge wenigstens ein in horizontaler Richtung sowie ein in vertikaler Richtung ansprechender Sensor zur Verfügung, so daß man wenigstens zwei Komponenten der Impulse für die Auswertung zugrunde legen kann. Eine weitere Differenzierung der Auswertung wird dadurch gegeben, daß, abweichend vom Stand der Technik, die von der Herztätigkeit ausgelösten Signale nicht mehr herausgefiltert werden, sondern der gleichzeitigen Überwachung unterliegen. Insofern kommt der Signalfrequenz und Signalform eine differenzierende Bedeutung zu, da sie es gestattet, die von der Atmungstätigkeit und der Herztätigkeit herrührenden Impulse voneinander zu trennen. Für jeden dieser Vorgänge kommt es mithin zur Registrierung wenigstens zweier Impulskomponenten, von denen einer in einer horizontalen Ebene und der zweite vertikal ausgerichtet ist. Besonders zweckmäßig ist die Ausrichtung zweier Sensoren in der horizontalen Ebene, die dann ihrerseits wiederum rechtwinklig zueinander stehen. Demgemäß erfaßte Impulskomponenten besitzen zusammen mit ihrer frequenzbedingten Differenzierung eine weitreichende Aussagefähigkeit in Richtung auf richtungsabhängige Impulse der Atmungs- und Herztätigkeit.

Die erfindungsgemäße Vorrichtung läßt sich in unterschiedlicher Art und Weise ausführen. Um eine allseitige Beweglichkeit sicherzustellen, kann eine Aufhängung oberhalb des Schwerpunktes des Bettes oder auch eine Unterstützung desselben unterhalb seines Schwerpunktes in Frage kommen, wobei im erstgenannten Fall das Rückstellmoment bei Lageabweichungen auf Grund der Schwerpunktsverschiebung gegeben ist, wohingegen im zweigenannten Fall einer sogenannten instabilen Lagerung zusätzliche Mittel vorgesehen sind, die eine Rückstellung bewirken. Als derartige zusätzliche Mittel, die berührungsfrei arbeiten können, lassen sich beispielsweise gleichgepolte Magnete beim Bett und an einer ortsfesten Gegenstelle vorsehen, auf Grund welcher eine Normallage des Bettes eingehalten werden kann. Stattdessen kommen aber auch mit der Abstützung selbst zusammenwirkende Massen in Betracht, durch die es gelingt, den Schwerpunkt des gesamten Systems entsprechend zu verlagern, so daß von ihm wirkungsvolle Rückstellkräfte ausgehen können. Neben dem vertikal ausgerichteten Sensor findet in allen Fällen wenigstens ein horizontal ausgerichteter Sensor Anwendung, und es kommt lediglich darauf an, entsprechend Einzelmerkmalen der Erfindung die Sensoren so anzuordnen, daß sie aussagefähige, differenzierte Signale ermöglichen.

Eine Aufhängung oberhalb des Schwerpunktes des Bettes wird zweckmäßig so ausgeführt, daß in der Wirkungsrichtung des Schwerpunktes der vertikal ausgerichtete Sensor angeordnet ist, und daß mit horizontalem Abstand dieser Wirkungsrichtung der oder die übrigen Sensoren mit der Aufhängung bzw. mit dem Bett bei Auslenkung des letzteren aus seiner Normallage zusammenwirken.

Das Bett läßt sich auch kardanisch lagern, in welchem Falle der vertikal ausgerichtete Sensor bei der Abstützung einer der Lagerachsen Verwendung findet, während der oder die übrigen Sensoren auf Kanten des Bettes oder Rahmenteile ausgerichtet werden. Die letztgenannte Anordnung hat den Vorteil, daß das Bett von oben frei zugänglich bleibt.

Ein ähnlicher Vorteil läßt sich auch bei einer Lagerung unterhalb des Schwerpunktes des Bettes verwirklichen, indem eine bodenseitige Tragsäule des Bettes eine Lagerkugel durchsetzt, die auf Tragkugeln bewegt werden kann. Die Tragsäule ist zweckmäßig nach Durchgang durch die Lagerkugel mit einem Gegengewicht versehen. Der vertikal ausgerichtete Sensor läßt sich unmittelbar in der Tragsäule zwischen Lagerkugel und Bett anordnen, wohingegen die weiteren Sensoren an der Tragsäule vorgesehen sein können, sofern es sich z.B. um Elemente eines Hallgenerators, kondensators oder strahlungsreflektierende bzw. strahlungserzeugende Mittel handelt. Ortsfest zu den letztgenannten Sensoren sind dann beispielsweise Empfänger angeordnet, die das Ausmaß einer Auslenkung erkennen lassen.

Bei einer schwimmenden Lagerung oder einer ähnlich wirkenden Lagerung mittels einer das Bett unterstützenden Spiraldruckfeder besteht der vertikal ausgerichtete Sensor bei der Abstützung einer horizontalen Kante des Bettes, wohingegen die weiteren Sensoren auf eine vertikale Kante des Bettes ausgerichtet sind. Die Normallage wird stabilisiert, indem insbesondere die bereits vorerwähnten Magnetpole und ihnen entsprechende, gleichgerichtete Pole ortsfester Magnete vorgesehen sind. Besonders platzsparend und wirkungsvoll läßt sich das Bett mittels einer nach unten gewölbten Kugelkalotte lagern, in der sich ein massereicher Gewichtskörper befindet, die derart massereich ist, so daß das gesamte System seinen Schwerpunkt hinreichend nach unten verlagert, um stets eine Rückstellkraft zu erzeugen. Der vertikal ausgerichtete Sensor muß in diesem Falle in Nähe des Lagerpunktes der Kugelkalotte auf der ortsfesten Unterlage vorgesehen sein und auf den Lagerpunkt ausgerichtet werden. Die übrigen Sensoren lassen sich auf die Bettkanten ausrichten. Neben der leichten Zugänglichkeit des Bettes von allen Seiten ist die hinreichend schwere Masse innerhalb der Kugelkalotte leicht zu verwirklichen, so daß sich die gewünschte Schwerpunktsverschiebung des gesamten Systems ergibt.

In allen Fällen eines vertikal ausgerichteten Sensors ist dieser auch für statische Messungen insbesondere des Gewichtes von Neugeborenen geeignet.

Die Erfindung sei weiterhin anhand der sich auf Ausführungsbeispiele beziehenden Zeichnungen veranschaulicht. Darin zeigen:

| | |
|---|---|
| Figur 1 | eine erste Anordnung des Bettes und der Sensoren, |
| Figur 2 | eine abgewandelte Ausführungsform der Anordnung nach Figur 1, |
| Figur 3 | einen vergrößerten Ausschnitt des Bereiches III der Ausführungsform nach Figur 2, |
| Figur 4 | eine kardanische Lagerung des Bettes mit Anbringung der Sensoren, |
| Figur 5 | die Anordnung des Bettes mittels einer Lagerkugel und die Lage der Sensoren, |
| Figur 6 | eine Abwandlungsform der Lagerkugel gemäß Figur 5, |
| Figur 7 | die seitliche Ansicht einer schwimmenden Lagerung, |
| Figur 8 | eine Draufsicht entsprechend Figur 7, |
| Figur 9 | eine abgewandelte Ausführungsform der Anordnung gemäß Figur 7 und 8, |
| Figur 10 | eine magnetische Lagestabilisierung, |
| Figur 11 | eine Lageanordnung des Bettes mit einer Kugelkalotte **und** |

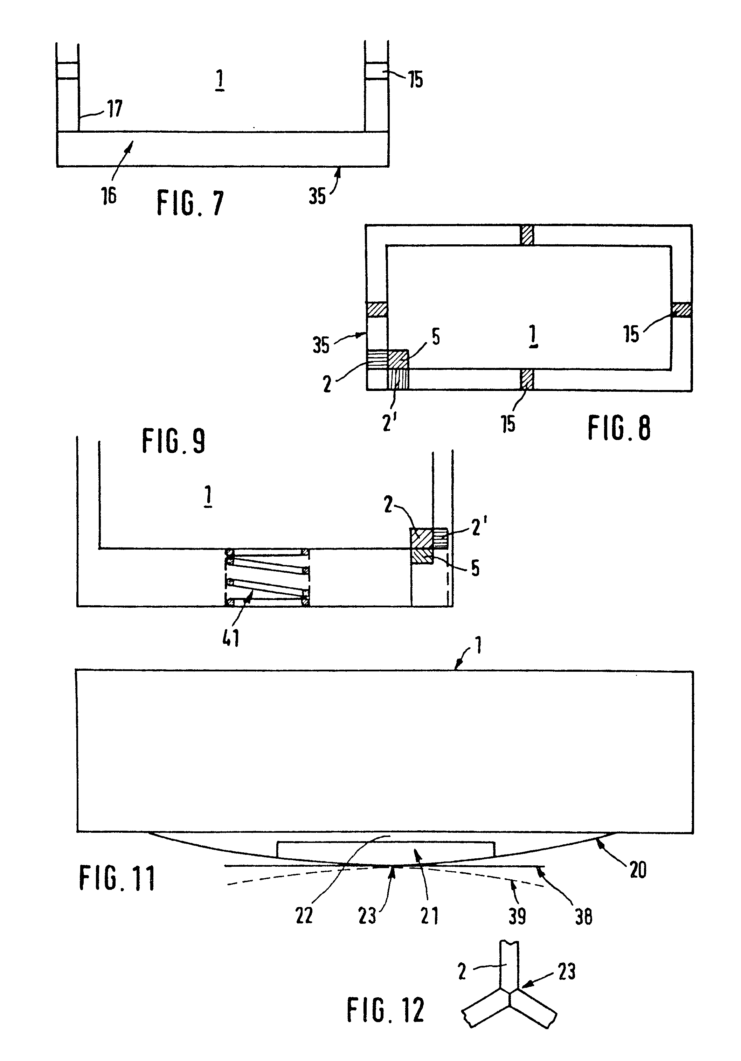

Figur 12     die Anordnung der horizontal ausgerichteten Sensoren für eine Lagerung gemäß Figur 11.

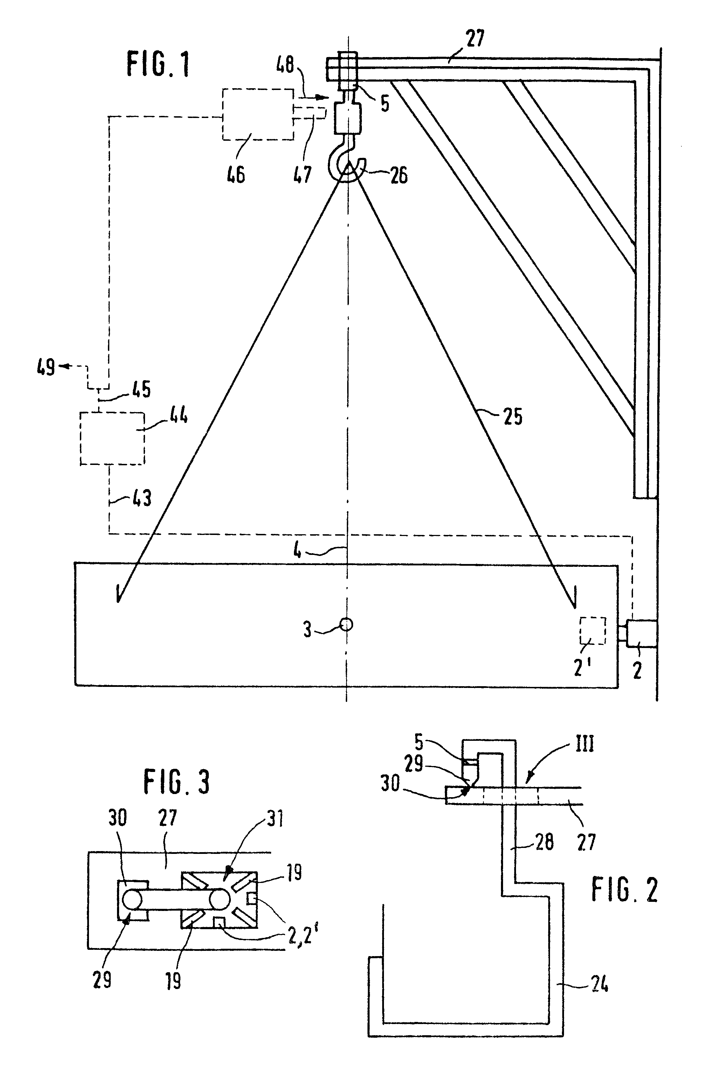

Das Bett 1 ist entsprechend Figur 1 oberhalb seines Schwerpunktes 3 mittels Gurten 25 am Haken 26 eines starren Wandarmes 27 aufgehängt. In diese Aufhängung ist der vertikal ausgerichtete Sensor 5 einbezogen, so daß er in der Wirkungsrichtung 4 des Schwerpunktes 3 des Bettes 1 liegt. Damit werden die in vertikaler Richtung ausgeübten Kräfte erfaßt.

Die rechte Seitenwand des Bettes 1 ist mittels eines Zwischengliedes an einer ortsfesten Wand abgestützt, wobei in diese Abstützung der weitere, horizontal ausgerichtete Sensor 2 einbezogen ist. Man erkennt noch einen gestrichelt dargestellten weiteren Sensor 2', der die im Winkel von 90° zur Ausrichtung des Sensors 2 gerichteten Kräfte aufzunehmen gestattet. Sowie das Bett eine Ablenkung aus der Normallage erfährt, kehrt es unter der Einwirkung des von seinem Schwerpunkt 3 ausgeübten Momentes selbsttätig in seine Normallage zurück.

Damit verwirklicht die Anordnung nach Figur 1 das Wesen der Erfindung, indem einerseits die vertikale Komponente und andererseits zwei zueinander senkrecht stehende Horizontalkomponenten von Impulsen erfaßt werden, die sich auf Grund der Herz- und Atmungsreaktionen einer im Bett 1 befindlichen Person ergeben.

Die Ausführungsform nach Figur 2 verwendet anstatt Gurte einen Tragrahmen 24, bei dem ein hochstehender Rahmenarm 28 bis zur Mitte des Bettes hin verkröpft ist, so daß sich eine Lagerspitze 29 ergibt, über welche der Tragrahmen 24 mit dem Bett am vorstehenden Wandarm 27 an der Lagerstelle 30 abgestützt ist. In die Lagerspitze 29 ist der vertikal ausgerichtete Sensor 5 integriert. Der Wandarm 27 ist, wie Figur 3 zeigt, mit einem Ausschnitt 31 ausgebildet, in dessen Wandung sich die weiteren, horizontal ausgerichteten Sensoren 2, 2' befinden, die unter einem Winkel von 90° auf den Rahmenarm 28 ausgerichtet sind und entsprechend einer von dort möglichen Reflektion von Sensorstrahlen Auslenkungen des Rahmenarmes 28 aus seiner Normallage ermitteln, indem beispielsweise der reflektierte Sensorstrahl auf Fotodiodenzeilenkameras gerichtet wird. Im übrigen können die Sensoren, soweit sie in Bauteile integriert sind, als auf Zug- oder Druckspannung ansprechende Widerstände ausgestaltet sein. Figur 3 zeigt weiterhin die unter dem Gesichtspunkt einer stabilen Lagerung ausgebildete Lagerstelle 30. Ebenfalls erkennt man die Pole ortsfester Magnete 19, die derart aufeinander ausgerichtet sind, daß der Rahmenarm 28 in seiner Normallage stabilisiert wird, was insoweit über die Stabilisierung auf Grund der Schwerpunktslage noch eine zusätzliche Stabilisierungskraft wirksam ist.

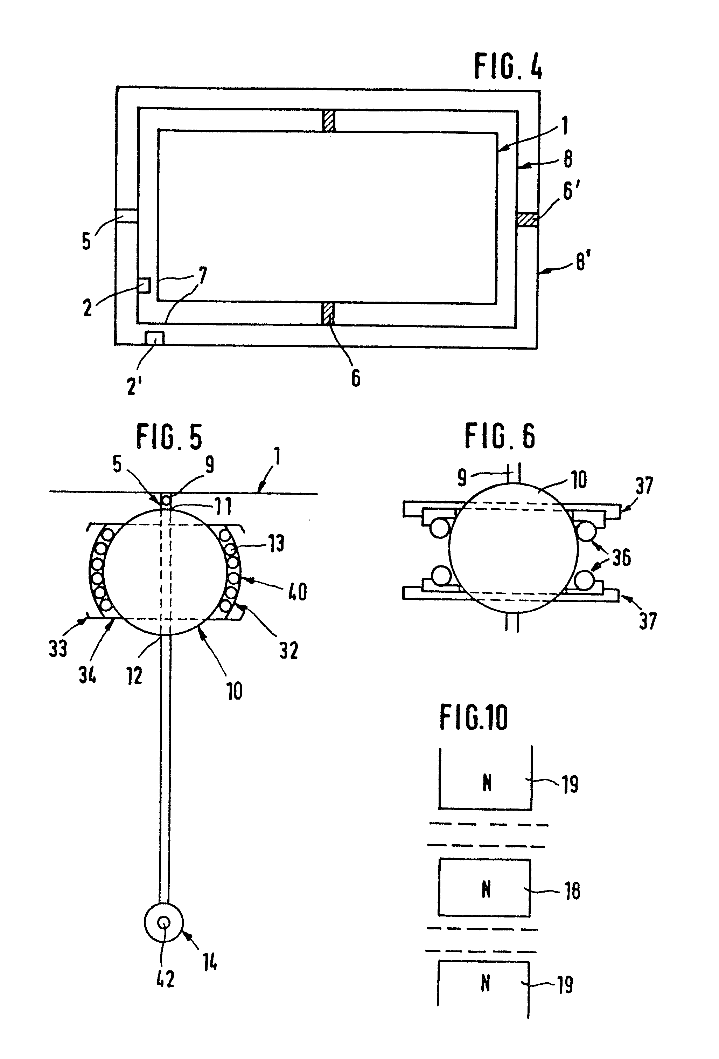

Die in Figur 4 dargestellte kardanische Aushängung sieht zwei senkrecht zueinander angeordnete Lagerachsen 6 bzw. 6' vor, von denen die Lagerachse 6 des Bettes 1 unmittelbar im Rahmen 8 lagert, der seinerseits mittels des Lagers 6' in einem ortsfesten Rahmen 8' gelagert ist. Unterhalb der Achse des Lagers 6' befindet sich der in vertikaler Richtung ausgerichtete Sensor 5, wohingegen zwei weitere, horizontal auf die Kanten 7 ausgerichtete Sensoren 2, 2' einerseits Auslenkungen des Rahmens 8 und andererseits Auslenkungen des Bettes 1 erfassen.

Nach Figur 5 ist das Bett 1 mittels einer Tragsäule 9 gelagert, welche eine Lagerkugel 10 in den Durchstoßstellen 11 und 12 durchsetzt. Die Lagerkugel 10 ist in einem Kugelkäfig 40 mit Tragkugeln abgestützt, dessen äußere Kugelschale 32 von einer Stützschale 33 gehalten ist, welche gegenüber der Lagerkugel 10 mit einer umlaufenden Dichtung 34 versehen ist. Der untere Teil der Tragsäule 9 trägt ein Gegengewicht 14, welches derart angeordnet ist, daß es die Normallage des Bettes 1 stabilisiert, wobei der Reibungswiderstand zwischen der Lagerkugel 10 und den Tragkugeln 13 leicht überwunden wird. Zwischen der oberen Durchstoßstelle 11 der Tragsäule 9 durch die Lagerkugel 10 und dem Boden des Bettes 1 befindet sich der vertikal ausgerichtete Sensor 5. Das Gegengewicht 14 trägt einen Reflektor 42 auf den Sensorstrahlen einer zeichnerisch nicht dargestellten Lichtquelle gerichtet sind,so daß Auslenkungen des Bettes 1 von horizontal ausgerichteten Sensoren erfaßbar sind. Bei der Abwandlung gemäß Figur 6 finden lediglich ein oberer und ein unterer Kugelkranz 36 Anwendung, welche Kugelkränze in der Halterung 37 befestigt sind. Mithin wirkt die Lagerung nach den Figuren 5 und 6 wie ein Pendel, welches die Bewegungen des Bettes aus seiner Normallage kompensiert. Die Stabilisierung der Lager auf Grund des Schwerpunktes gestattet eine Kombination dieser Art der Lagerung mit der gemäß Figur 1.

Die schwimmende Lagerung nach Figur 7 sieht die Anordnung des Bettes 1 in einer äußeren Schale 35 vor, die beispielsweise eine Flüssigkeit 16 aufnimmt. Der Boden des Bettes 1 trägt dabei zugleich eine Dichtung. Gegenüber der Schale 12 ist der Rahmen des Bettes 1 auf Abstand gehalten, welchem Zweck die Abstützungen 15 dienen. Diese sind in den Figuren 7 und 8 nur schematisch widergegeben. Sie können, ähnlich nach dem Schema der Figur 3, magnetisch verwirklicht werden, indem insbesondere Magnetpole 18 gemäß Figur 10 am Bett angeordnet sind, während ortsfeste Magnete 19 gleichgerichtet angeordnet sind, so daß ihre Pole die Pole 18 auf Abstand zu halten suchen. Wie vor allem Figur 8 erkennen läßt, lassen sich vertikal ausgerichtete Sensoren 5 zwischen dem Boden des Bettes 1 und der Lagerschale anbringen, wohingegen horizontal ausgerichtete Sensoren 2, 2' so angeordnet sind, daß sie auf die vertikalen Kanten 17 des Bettes 1 weisen und demgemäße Lageveränderungen aufnehmen können.

Zu einer ähnlichen Anordnung kommt man nach Figur 9, in welchem Fall das Bett 1 auf einer Spiraldruckfeder 41 angeordnet ist. Man erkennt wiederum den vertikal ausgerichteten Sensor 5 sowie die beiden horizontal ausgerichteten Sensoren 2 und 2'.

Bei der Anordnung nach Figur 11 ist das Bett 1 auf einer Kugelkalotte 20 angeordnet, wobei sich zwischen dem Boden des Bettes 1 und der genannten Kugelkalotte ein Gewichtskörper 21 befindet, so daß sich eine Schwerpunktslage 22 zwischen Bett 1 und Gewichtskörper 21 ergibt, die das Bett in seiner Normallage stabilisiert. Bei der gemeinschaftlichen Schwerpunktslage findet zusätzlich noch das Gewicht der Kugelkalotte 20 Berücksichtigung, so daß letztere zweckmäßig aus einem schweren Material hergestellt wird. Die Kugelkalotte 20 stützt sich im Lagerpunkt 23 entweder auf den horizontalen Boden 38 oder auf einen entgegengesetzt gekrümmten Lagerelement 39 ab. In Nähe des Lagerpunktes 23 ist der hier nicht wiedergegebene vertikal ausgerichtete Sensor 5 vorgesehen, für dessen Ausrichtung die Wirkungslinie der gemeinsamen Schwerpunktslage 22 maßgeblich ist. Wie Figur 12 zeigt, ist weiterhin noch eine Anordnung von Sensoren 2 vorgesehen, die horizontal auf der Lagerstelle 23 auflagern, so daß sie unter einem Winkel von jeweils 120° gegeneinander angeordnet sind. Auslenkungen des Bettes nach der einen oder nach der anderen Richtung führen zur Belastung der Sensoren 2, so daß die von diesen ausgehenden Signale für die jeweiligen Impulskomponenten kennzeichnend sind.

Eine Anordnung des Gewichtskörpers läßt sich nach Größe und Lage zusätzlich auch unter dem Gesichtspunkt treffen, daß damit die Eigenschwingungsfrequenz der Gesamtanordnung verändert wird. Man kann hierdurch erreichen, daß störende Schwingungen der Gesamtanordnung vermieden werden, indem sie in einer derartigen Frequenz auftreten, die keine Verfälschung der zu messenden Atmungs- bzw. Herzsignale ergibt. Insofern verbessert eine Anordnung des Gewichtskörpers 21 die Verfügbarkeit zuverlässig verwertbarer Signale.

Der bzw. die Sensoren geben, wie es nach dem zugrunde gelegten Stand der Technik bekannt war, ihre Signale an Schaltungselemente ab, auf Grund welcher beim Ausbleiben der Signale eine Alarmeinrichtung betätigt wird, die es dem Pflegepersonal ermöglicht, beispielsweise bei Atemstillstand sofortige Gegenmaßnahmen einzuleiten. Die vorliegende Erfindung benutzt das einen Alarm auslösende Sekundärsignal zusätzlich, um eine automatische Weckeinrichtung zu betätigen. Zu diesem Zweck wird das genannte Sekundärsignal für die Einschaltung eines Antriebes verwendet, der das Bett in Bewegung versetzt, so daß insbesondere Kleinkinder hierdurch aufgeweckt und wieder normal zu atmen beginnen.

Selbst wenn sich die Normalisierung der Atmungstätigkeit hierdurch noch nicht wieder in vollem Maße erzielen läßt, wird wertvolle Zeit genutzt, bis das Pflegepersonal auf Grund des gleichzeitig ausgelösten Alarms zur Verfügung steht. Die Anordnung läßt sich im übrigen auch so treffen, daß durch den Antrieb nur eine einzelne Bewegung des Bettes ausgelöst wird. In diesem Falle werden die dann darauf abgegebenen Signale durch die Funktion des Antriebes nicht weiter gestört.

Die vorbeschriebene Anordnung ergibt sich aus Figur 1 durch die darin gestrichelt eingezeichnete Ergänzung. Demnach führt vom Sensor 2 ein Signal 43 zu einem Verstärker und Signalumwandler 44. Dieses, hier nur als Blockschaltbild dargestellte Bauteil ist als solches nach dem Stand der Technik bekannt. Es erzeugt bei für eine vorgegebene Zeit bestehendem Ausbleiben des Signales 43 ein Sekundärsignal 45. Dieses Sekundärsignal löst nach dem bekannten Stand der Technik eine Alarmeinrichtung 49 aus. Gemäß der Erfindung verläuft hierzu noch parallel eine Verbindung des Sekundärsignals 45 zu einem Antrieb 46, also beispielsweise zu einem Elektromotor, so daß dann, wenn ein Signal 45 besteht, dieser Antrieb eingeschaltet wird. Der Antrieb ist derart konstruiert, daß ein Stößel 47 bei eingeschaltetem Antrieb in Richtung des Pfeiles 48 ausfahren kann. Dabei drückt der Stößel 47 vor das zum Haken 26 gehörende, am Wandarm 27 aufgehängte Bauteil, so daß das Bett 1 in Bewegung gerät. Diese Bewegung kann bereits ausreichend sein, um einen Weckvorgang auszulösen und den vorübergehenden Atemstillstand zu beheben.

## Patentansprüche

1. Vorrichtung zur berührungsfreien Überwachung der Atmung und Herztätigkeit bei einer in einem Bett (1) ruhenden Person, insbesondere bei Kleinkindern, wobei von der zu überwachenden Person ausgehend mechanische Kraftimpulse auf das Bett übertragen werden und von dort auf einen in vertikaler Richtung ausgerichteten, auf mechanische, in vertikaler Richtung wirkenden Kräfte ansprechenden Sensor (5) einwirken, welcher Sensor (5) zwischen dem Bett und einem ortfesten Stelle angeordnet ist, dadurch gekennzeichnet,

daß das Bett (1) allseitig beweglich gelagert ist,

und daß das Bett bei Abweichungen aus seiner normalen Lage einer letztere wiederherstellenden Rückstellkraft unterliegt,

und daß für das Bett eine in horizontaler Richtung gerichtete Abstützung vorgesehen ist,

und daß wenigstens ein weiterer Sensor (2) zur Aufnahme Lageveränderungen des Bettes in gleicher horizontaler Richtung wie die Abstützung des Bettes angeordnet ist.

2. Vorrichtung nach Anspruch 1, dadurch gekennzeichnet, daß für das Bett (1) eine Einzelaufhängung oberhalb seines Schwerpunktes (3) besteht, in deren Wirkungsrichtung der vertikal ausgerichtete Sensor (5) angeordnet ist, und daß mit horizontalem Abstand dieser Wirkungsrichtung (4) der oder die übrigen Sensoren mit der Aufhängung bzw. mit dem Bett bei Auslenkung des letzteren aus seiner Normallage zusammenwirken.

3. Vorrichtung nach Anspruch 1, dadurch gekennzeichnet, daß das Bett (1) kardanisch gelagert ist, wobei der vertikal ausgerichtete Sensor (5) bei der Abstützung einer Lagerachse (6) vorgesehen ist und der bzw. die weiteren Sensoren (2) auf Kanten (7) des Rahmens (8) oder Bettes (1) ausgerichtet sind.

4. Vorrichtung nach Anspruch 1, dadurch gekennzeichnet, daß eine bodenseitige Tragsäule (9) des Bettes (1) eine Lagerkugel (1o) durchsetzt, die außerhalb ihrer oberen und unteren Durchstoßstellen (11,12) mittels Tragkugeln (13) gelagert ist, wobei der vertikal ausgerichtete Sensor (5) in der Tragsäule (9) zwischen Lagerkugel (1o) und dem Boden des Bettes (1) angebracht ist, während sich am unteren, die Tragkugel (1o) durchsetzenden Teil der Tragsäule (9) ein Gegengewicht (14) sowie der oder die weiteren Sensoren (2) befinden.

5. Vorrichtung nach Anspruch 1, dadurch gekennzeichnet, daß der vertikal ausgerichtete Sensor (5) bei der Abstützung (15) einer horizontalen Kante (7) des Bettes (1) besteht, während der oder die weiteren Sensoren (2) auf eine vertikale Kante des Bettes (1) ausgerichtet sind.

6. Vorrichtung nach Anspruch 5, dadurch gekennzeichnet, daß das Bett (1) schwimmend gelagert ist.

7. Vorrichtung nach Anspruch 5, dadurch gekennzeichnet, daß das Bett (1) auf einer es mittig unterstützenden Spiraldruckfeder angeordnet ist.

8. Vorrichtung nach den Ansprüchen 1, 2, 5, 6 und 7, dadurch gekennzeichnet, daß das Bett (1) mittels von seinen Rändern nach außen gerichteten Magnetpolen (19), denen Pole gleichgerichteter, ortsfester Magnete (19) entsprechen, in seiner Normallage gehalten ist.

9. Vorrichtung nach Anspruch 1, dadurch gekennzeichnet, daß das Bett (1) mittels einer an seinem Boden vorgesehenen und nach unten gewölbten Kugelkalotte (2o) gelagert ist, innerhalb welcher sich ein Gewichtskörper (21) derart befindet, daß auf Grund der gemeinsamen Schwerpunktslage (22) der vorgenannten Gegenstände (1,2o,21) bei Auslenkung des Bettes (1) aus seiner Normallage die Rückstellung erfolgt, wobei der vertikal ausgerichtete Sensor (5) in Nähe des Lagerpunktes (23) vorgesehen und auf diesen ausgerichtet ist, während der oder die weiteren Sensoren (2) auf die Bettkanten (7,17) ausgerichtet sind.

10. Vorrichtung nach den Ansprüchen 1 bis 9, wobei die durch den Sensor erzeugten Signale in einer Schaltung derart ausgewertet werden, daß beim Ausbleiben der Signale durch ein Sekundärsignal eine Alarmeinrichtung betätigt wird, dadurch gekennzeichnet, daß parallel zur Alarmeinrichtung ein Antrieb eingeschaltet wird, der das Bett derart in Bewegung versetzt, daß die darin ruhende Person aufgeweckt wird.

11. Vorrichtung nach Anspruch 10, dadurch gekennzeichnet, daß der Antrieb lediglich für eine einzelne Weckbewegung des Bettes durch das Sekundärsignal einschaltbar ist.

**Claims**

1. Apparatus for monitoring the respiratory and cardiac activity of a person resting in a bed (1), specifically in the case of small children, with pulses emitted by the monitored person acting on a vertically aligned sensor (5), **characterized by** the fact

    that the bed (1) is supported in a manner permitting its movement in any direction;

    and that a reset force acting on said bed will return it to its normal position if and when it is displaced from said normal position;

    and that there is provided at least one other sensor (2 horizontally aligned near a support extending in the same direction.

2. Apparatus as claimed in Claim 1, **characterized by** the fact that there is provided for said bed (1), at a point above its center of gravity (3), a single suspension means in whose effective direction there is disposed the vertically aligned sensor (5), and that, as the horizontal distance in said effective direction (4) varies, the remaining sensor(s) cooperate with the suspension means and/or with the bed if and when the latter is displaced from its normal position.

3. Apparatus as claimed in Claim 1, **characterized by** the fact that the led (1) rests in a gimbal mount, with the vertically aligned sensor (5) being disposed near the support of one mounting axis (6) while the other sensor(s) (2) are aligned on edges (7) of the frame (8) or bed (1).

4. Apparatus as claimed in Claim 1, **characterized by** the fact that a floor support column (9) of the bed (1) passes through a spherical support means (10) resting in a plurality of support balls (13) arranged outside the upper and lower penetration points of said support column, with the vertically aligned sensor (9) being attached inside the support column (9) between said spherical support means (10) and the bottom of the bed (1), while at the lower part of the support column (9) passing through the spherical support means (10) there is provided a counterweight (14) together with the other sensor(s) (2).

5. Apparatus as claimed in Claim 1, **characterized by** the fact that the vertically aligned sensor (5) is attached near the support (15) of one horizontal edge (7) of the bed (1) whereas the other sensor(s) (2) are aligned with a vertical edge of the bed (1).

6. Apparatus as claimed in Claim 5, **characterized** by the fact that the bed (1) rests in a floating suspension system.

7. Apparatus as claimed in Claim 5, **characterized by** the fact that the bed (1) rests on a helical compression spring supporting it in its middle.

8. Apparatus as claimed in Claims 1, 2, 5, 6 and 7, **characterized by** the fact that the bed (1) is maintained in its normal position by means of magnetic poles (19) which, being directed outwardly from its edges, correspond to the poles of stationary magnets (19) acting in the same direction.

9. Apparatus as claimed in Claim 1, **characterized** by the fact that the bed (1) rests on a downwardly curved body (20) exhibiting the shape of a spherical sector, said body containing a weight (21) acting in such a manner that the bed (1), when displaced from its normal position, is returned into the latter as a result of the common center of gravity (22) of the aforementioned items (1, 20, 21) with the vertically aligned sensor (5) being attached near the support point (23) and aligned with the latter, whereas the other sensor(s) are aligned with the edges (7, 17) of the bed.

10. Apparatus as claimed by Claims 1 through 9 wherein the signals generated by the sensor are analyzed by a corresponding circuit in such a manner that, when these signals cease, a secondary signal triggers an alarm system, said arrangement being **characterized by** the fact that, together with the alarm system, a drive system is activated to move the bed so as to wake up the person resting in said bed.

11. Apparatus as claimed in Claim 10, **characterized by** the fact that the drive system can only be activated by the secondary signal so as to perform a single wake-up movement.

**Revendications**

1. Dispositif de surveillance sans contact de la respiration et du fonctionnement cardiaque de personnes allongées dans un lit (1), en particulier d'enfants en bas-âge, dans lequel les impulsions de puissance mécaniques émanant de la personne objet de la surveillance sont transmises au lit, d'où elles agissent sur un capteur (5) orienté verticalement réagissant aux forces mécaniques et agissant verticalement, lequel capteur (5) est implanté entre le lit et un point fixe,
**caractérisé en ce que**
le lit (1) est logé de manière mobile de tous les côtés, qu'i est soumis, en cas de divergence par rapport à sa position normale, à une force de rappel rétablissant ladite position, et qu'un capteur supplémentaire (2) destiné à l'enregistrement de la modification du lit est implanté dans le même sens horizontal que le support du lit.

2. Dispositif selon la revendication 1,
**caractérisé en ce qu'il**
existe pour le lit, au-dessus de son centre de gravité (3), une suspension individuelle dans la direction effective de laquelle est implanté le capteur (5) orienté verticalement, que le ou les autre(s) capteurs(s) agissent ensemble avec le lit en cas de déplacement de ce dernier de sa situation normale avec un écartement horizontal de ce sens effectif.

3. Dispositif selon la revendication 1,
   **caractérisé en ce que**
   le lit (1) est logé de manière cardanique, le capteur à orientation verticale (5) étant prévu sur le support d'une languette de logement (6), et que le ou les autre(s) capteur(s) (2) sont orientés sur des bords (7) du cadre (8) ou du lit (1).

4. Dispositif selon la revendication 1,
   **caractérisé en ce qu'**une
   colonne de support (9) du lit (1) placé sous le sommier de ce dernier est traversée par une bille de roulement (10) qui est logée au-dessus et en-dessous de ses points de traversée (11, 12) au moyen de billes de support (13), le capteur orienté verticalement (5) étant imlpanté dans la colonne de support (9) entre la bille de roulement (10) et le sommier du lit (1), cependant que se trouve, sur la partie traversant la bille de support (10) de la colonne portante (9) un contrepoids (14) ainsi que le ou les autre(s) capteur(s) (2).

5. Dispositif selon la revendication 1,
   **caractérisé en ce que**
   le capteur orienté verticalement (5) existe sur le support (15) d'un bord horizontal (7) du lit, cependant que le ou les autre(s) capteur(s) (2) sont alignés sur un bord vertical du lit.

6. Dispositif selon la revendication 1,
   **caractérisé en ce que**
   le lit (1) est logé de manière flottante.

7. Dispositif selon la revendication 1,
   **caractérisé en ce que**
   Le lit (1) est implanté sur un ressort de pression en spirale le soutenant en son axe.

8. Dispositif selon les revendications 1, 2, 5, 6 et 7,
   **caractérisé en ce que**
   le lit (1) est maintenu dans sa position normale au moyen de pôles magnétiques (19) orientés vers l'extérieur par rapport à ses bords, et dont les pôles correspondent à des aimants redressés fixes (19).

9. Dispositif selon la revendication 1,
   **caractérisé en ce qu'**le
   lit (1) est logé au moyen d'une calotte sphérique (20) prévue sur son sommier et bombée vers le bas, et dans laquelle se trouve un corps pesant (21) tel que le lit (1) soit rappelé en sa position normale du fait du centre de gravité commun des objets précités (1, 20, 21) en cas de déplacement, le capteur à orientation verticale (5) étant prévu à proximité du point de logement (23), cependant que le ou les autre(s) capteur(s) (2) sont orientés vers les bords du lit (7, 17).

10. Dispositif selon les revendications 1 à 9, pour lequel les signaux émis par le capteur sont analysés par un circuit de manière telle qu'en l'absence des signaux un signal secondaire manoeuvre un équipement d'alarme,
    **caractérisé en ce que,**
    parallèlement à l'équipement d'alarme, un entraînement mettant le lit en mouvement de manière telle que la personne y allongée est réveillée.

11. Dispositif selon la revendication 10,
    **caractérisé en ce que**
    l'entraînement ne peut être commuté par le signal secondaire que pour un seul mouvement de réveil du lit.

FIG.1

FIG. 3

FIG. 2

FIG. 4

FIG. 5

FIG. 6

FIG. 10

FIG. 7

FIG. 8

FIG.9

FIG. 11

FIG. 12